# EUROPEAN PATENT APPLICATION

(11) **EP 4 400 531 A1**
(43) Date of publication of application: **17.07.2024**
(21) Application number: 23836706.4
(22) Date of filing: 01.06.2023
(51) Int. Cl.: C08G 64/30

(54) **MONOMER COMPOSITION FOR SYNTHESIZING RECYCLED PLASTIC, METHOD FOR PREPARING SAME, AND RECYCLED PLASTIC AND MOLDED ARTICLES USING SAME**

(30) Priority: 14.11.2022 KR 20220151487; 24.05.2023 KR 20230067219
(71) Applicant: LG Chem, Ltd., Seoul 07336 (KR)
(72) Inventor: KIM, Jeongnam, Daejeon 34122 (KR); BAE, Jungmoon, Daejeon 34122 (KR); LEE, Ki Jae, Daejeon 34122 (KR); LEE, Jeongbin, Daejeon 34122 (KR); HWANG, Jihwan, Daejeon 34122 (KR); HONG, Mooho, Daejeon 34122 (KR)
(74) Representative: Goddar, Heinz J.
(86) International application number: PCT/KR2023/007534
(87) International publication number: WO 2024/106653

(57) **Abstract**

The present invention relates to a monomer composition for synthesizing recycled plastic that can realize excellent color quality even though it is recovered through recycling by chemical decomposition of a polycarbonate-based resin, and allows improvement of efficiency in the recovery process, a preparation method thereof, and a recycled plastic and a molded product using the same.

## Description

### [TECHNICAL FIELD]

### CROSS-REFERENCE TO RELATED APPLICATION(S)

This application claims the benefit of Korean Patent Application No. 10-2022-0151487 filed on November 14, 2022 and Korean Patent Application No. 10-2023-0067219 filed on May 24, 2023 in the Korean Intellectual Property Office, the entire contents of which are incorporated herein by reference.

The present invention relates to a monomer composition for synthesizing recycled plastic that can realize excellent color quality even though it is recovered through recycling by chemical decomposition of a polycarbonate-based resin, and allows improvement of efficiency in the recovery process, a preparation method thereof, and a recycled plastic and molded product using the same.

### [BACKGROUND ART]

Polycarbonate is a thermoplastic polymer and is a plastic having excellent characteristics such as excellent transparency, superior ductility, and relatively low production costs.

Although polycarbonate is widely used for various purposes, environmental and health concerns during waste treatment have been continuously raised.

Currently, a physical recycling method is carried out, but in this case, a problem accompanying the deterioration of the quality occurs, and thus, research on the chemical recycling of polycarbonate is underway.

Chemical decomposition of polycarbonate refers to obtaining an aromatic diol compound as a monomer (e.g., bisphenol A (BPA)) through decomposition of polycarbonate, and then utilizing it again in polymerization to obtain a high-purity polycarbonate.

For such a chemical decomposition, thermal decomposition, hydrolysis, and alcohol decomposition are typically known. Among these, the most common method is alcohol decomposition using a base catalyst, but in the case of methanol decomposition, there is a problem that methanol is used which is harmful to the human body, and in the case of ethanol, there is a problem that high temperature and high pressure conditions are required and the yield is not high.

In addition, although an alcohol decomposition method using an organic catalyst is known, it is disadvantageous in terms of economics.

### [DETAILED DESCRIPTION OF THE INVENTION]

### [Technical Problem]

It is an object of the present invention to provide a monomer composition for synthesizing recycled plastic that can realize excellent color quality even though it is recovered through recycling by chemical decomposition of a polycarbonate-based resin, and allows improvement of efficiency in the recovery process.

It is another object of the present invention to provide a method for preparing the monomer composition for synthesizing recycled plastic, and a recycled plastic and a molded product using the monomer composition for synthesizing recycled plastic.

### [Technical Solution]

In order to achieve the above object, provided herein is a monomer composition for synthesizing recycled plastic, which comprises an aromatic diol compound, wherein a color coordinate b* is 1.8 or less, wherein an APHA color value measured according to ASTM D 1209 is 30 or less, and wherein the monomer composition is recovered from a polycarbonate-based resin.

Also provided herein is a method for preparing a monomer composition for synthesizing recycled plastic, the method comprising the steps of: subjecting a polycarbonate-based resin to a depolymerization reaction; separating a carbonate precursor from a depolymerization reaction product; and purifying the depolymerization reaction product from which the carbonate precursor has been separated, wherein the purifying the depolymerization reaction product from which the carbonate precursor has been separated comprises adding a hydrophilic reducing agent to the depolymerization reaction product from which the carbonate precursor has been separated; and adding an adsorbent to the depolymerization reaction product from which the carbonate precursor has been separated to perform adsorption purification, and then removing the adsorbent.

Further provided herein is a recycled plastic comprising a reaction product of the monomer composition for synthesizing recycled plastic and a comonomer.

Further provided herein is a molded product comprising the recycled plastic.

Below, a monomer composition for synthesizing recycled plastic, a preparation method thereof, and a recycled plastic, and molded product using the same according to specific embodiments of the present invention will be described in more detail.

Unless explicitly stated herein, the technical terms used herein are for the purpose of describing specific embodiments only and is not intended to limit the scope of the invention.

The singular forms "a," "an" and "the" used herein are intended to include plural forms, unless the context clearly indicates otherwise.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

### 1. Monomer Composition for Synthesizing Recycled Plastic

According to one embodiment of the present invention, there can be provided a monomer composition for synthesizing recycled plastic, which comprises an aromatic diol compound, wherein a color coordinate b* is 1.8 or less, wherein an APHA Color value measured according to ASTM D 1209 is 30 or less, and wherein the monomer composition is recovered from a polycarbonate-based resin.

The present inventors have found through experiments that although the monomer composition for synthesizing recycled plastics of the one embodiment was recovered through recycling by chemical decomposition of the polycarbonate-based resin, the aromatic diol compound, which is the main recovery target, can realize excellent color quality, thereby capable of realizing excellent physical properties in the synthesis of polycarbonate-based resins using the same, and completed the present invention.

In particular, the monomer composition for synthesizing recycled plastic according to one embodiment has technical advantages that among the preparation processes described below, the amount of organic solvent used in the adsorption purification process can be reduced, and also the amount of water used in the recrystallization process can be reduced, thereby improving the efficiency of the process and realizing excellent color quality.

Further, the present invention may have technical features and advantages that the first composition including the aromatic diol compound can be obtained in high purity through recycling by chemical decomposition of a polycarbonate-based resin, and at the same time, the second composition including diethyl carbonate, which is a high value-added by-product, can also be obtained.

Specifically, the monomer composition for synthesizing recycled plastic of the one embodiment is characterized by being recovered from a polycarbonate-based resin. That is, this means that as a result of performing the recovery from the polycarbonate-based resin in order to obtain the monomer composition for synthesizing recycled plastics of the one embodiment, the monomer composition for synthesizing recycled plastics containing the aromatic diol compound is obtained together.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate precursor. When it contains one carbonate repeating unit obtained by using only one aromatic diol compound and one carbonate precursor, a homopolymer can be synthesized. In addition, when one aromatic diol compound and two or more carbonate precursors are used as the monomer, or two or more aromatic diol compounds and one carbonate precursor are used, or one or more other diols is used in addition to the one aromatic diol compound and the one carbonate precursor to contain two or more carbonates, a copolymer can be synthesized. The homopolymer or copolymer can include all of lowmolecular compounds, oligomers, and polymers depending on the molecular weight range.

Further, the monomer composition for synthesizing recycled plastics of the one embodiment may include an aromatic diol compound. Specific examples of the aromatic diol compound include bis(4-hydroxyphenyl)methane, bis(4-hydroxyphenyl)ether, bis(4-hydroxyphenyl)sulfone, bis(4-hydroxyphenyl)sulfoxide, bis(4-hydroxyphenyl)sulfide, bis(4-hydroxyphenyl)ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or a mixture of two or more thereof, and the like. Preferably, the aromatic diol compound of the monomer composition for synthesizing recycled plastics of the one embodiment may be 2,2-bis(4-hydroxyphenyl)propane (bisphenol A).

The aromatic diol compound is characterized by being recovered from the polycarbonate-based resin used for recovering the monomer composition for synthesizing the recycled plastic. That is, this means that as a result of performing the recovery from the polycarbonate-based resin in order to obtain the monomer composition for synthesizing recycled plastics of the one embodiment, an aromatic diol compound is also obtained together. Therefore, apart from the recovery from the polycarbonate-based resin in order to prepare the monomer composition for synthesizing recycled plastics of the one embodiment, the case where a novel aromatic diol compound is added from the outside is not included in the category of aromatic diol compound of the present invention.

Specifically, "being recovered from the polycarbonate-based resin" means being obtained through a depolymerization reaction of the polycarbonate-based resin. The depolymerization reaction can be carried out under acidic, neutral or basic conditions, and particularly, the depolymerization reaction can proceed under basic (alkaline) conditions. Particularly, the depolymerization reaction can be preferably carried out in the presence of an ethanol solvent, as will be described later.

Meanwhile, the monomer composition for synthesizing recycled plastics of the one embodiment may have a color coordinate b* value of 1.8 or less, or 1.65 or less, or 0.1 or more, or 1.0 or more, or 0.1 to 1.8, or 1.0 to 1.8, or 0.1 to 1.65, or 1.0 to 1.65, or 1.6 to 1.7, or 1.6 to 1.65, or 1.65 to 1.7

Further, the monomer composition for synthesizing recycled plastics of the one embodiment may have a color coordinate L* of 95.5 or more, or 96 or more, or 100 or less, or 95.5 to 100, or 96 to 100, or 95.6 to 96.7, or 95.6 to 96, or 96 to 96.7.

Further, the monomer composition for synthesizing recycled plastics of the one embodiment may have a color coordinate a* of -0.7 to -0.2, or -0.6 to -0.2, or -0.5 to - 0.2, or -0.4 to -0.2, or -0.7 to -0.3 , or -0.6 to -0.3, or -0.5 to -0.3, or -0.4 to -0.3, or -0.7 to -0.5, or -0.6 to -0.5.

As used herein, the "color coordinates" means coordinates in the CIE Lab color space, which are color values defined by CIE (Commossion International de l'Eclairage), and an arbitrary position in the CIE color space may be represented by three coordinate values, i.e., L*, a*, and b*.

Here, the L* value represents brightness, when L*=0, it represents black, and when L*=100, it represents white. In addition, the a* value represents a color having a corresponding color coordinate that leans toward one of pure red and pure green, and the b* value represents a color having a corresponding color coordinate that leans toward one of pure yellow and pure blue.

Specifically, the a* value is in the range of -a to +a. A maximum value of a* (a* max) represents pure red, and a minimum value of a* (a* min) represents pure green. Further, the b* value is in the range of -b to +b. A maximum value of b* (b* max) represents pure yellow, and a minimum value of b* (b* min) represents pure blue. For example, a negative b* value represents a color leaning toward pure blue, and a positive b* value represents color leaning toward pure yellow. When comparing b*=50 with b*=80, b*=80 is closer to pure yellow than b*=50.

When the color coordinate b* value of the monomer composition for synthesizing recycled plastics of the one embodiment excessively increases to more than 1.8, the color coordinate L* value excessively decreases to less than 95.5, or the color coordinate a* value deviates from more than -0.2 or less than -0.7, the monomer composition for synthesizing recycled plastics of the one embodiment deteriorates in the color characteristics. Examples of the method for measuring the color coordinates L*, a*, b* values of the monomer composition for synthesizing recycled plastics of the one embodiment are not particularly limited, and various color characteristic measurement methods in the field of plastics can be applied without limitation.

However, the color coordinates L*, a*, and b* values of the monomer composition for synthesizing recycled plastics of the one embodiment can be measured in reflection mode using HunterLab UltraScan PRO Spectrophotometer as an example.

Meanwhile, the monomer composition for synthesizing recycled plastics of the one embodiment may have an APHA Color value of 30 or less, or 29 or less, or 28 or less, or 25 or less, or 22 or less, or 0 or more, or 1 or more, or 0 to 30, or 0 to 29, or 0 to 28, or 0 to 25, or 0 to 22, or 1 to 30, or 1 to 29, or 1 to 28, or 1 to 25, or 1 to 22, or 22 to 28, or 22 to 25, or 25 to 28, as measured according to ASTM D 1209.

Examples of the method for measuring the APHA Color of the monomer composition for synthesizing recycled plastics of the one embodiment are not particularly limited, and for example, it can be measured according to the ASTM D 1209 test method using HunterLab UltraScan PRO Spectrophotometer device.

The APHA Color is a value obtained from a color comparison between water having no color and a PtCo solution having a yellow color. A value closer to 0 indicates colorless, and a value closer to 500 indicates yellow. Therefore, as the APHA Color of the monomer composition for synthesizing recycled plastic of the one embodiment decreases to 30 or less, it is possible to realize transparent color properties in the synthesis of a polycarbonate-based resin using the same.

Meanwhile, when the APHA Color of the monomer composition for synthesizing recycled plastic of one embodiment excessively increases to more than 30 or the like, there is a limit in that it is difficult to synthesize a colorless and transparent polycarbonate-based resin due to yellowing.

Meanwhile, the monomer composition for synthesizing recycled plastics according to one embodiment may further include a hydrophilic reducing agent. The hydrophilic reducing agent is soluble in water and can be used as a bleaching agent that removes a color by changing colored substances. Examples of the hydrophilic reducing agent are not particularly limited, and various hydrophilic reducing bleaching agents generally known in the art may be applied without limitation, but sodium dithionite (SDT) may be mentioned as an example.

Meanwhile, in the monomer composition for synthesizing recycled plastics of the one embodiment, diethyl carbonate can be obtained as a by-product. The diethyl carbonate is characterized by being recovered from the polycarbonate-based resin used in the recovery of the monomer composition for synthesizing recycled plastics of the one embodiment.

That is, this means that as a result of performing the recovery from the polycarbonate-based resin in order to obtain the monomer composition for synthesizing recycled plastics of the one embodiment, dimethyl carbonate is also obtained together. Therefore, apart from the recovery from the polycarbonate-based resin in order to prepare the monomer composition for synthesizing recycled plastics of the one embodiment, the case where novel dimethyl carbonate is added from the outside is not included in the category of dimethyl carbonate of the one embodiment.

Specifically, "being recovered from the polycarbonate-based resin" means being obtained through a depolymerization reaction of the polycarbonate-based resin. The depolymerization reaction can be carried out under acidic, neutral or basic conditions, and particularly, the depolymerization reaction can proceed under basic (alkaline) conditions. Particularly, the depolymerization reaction can be preferably carried out in the presence of an ethanol solvent, as will be described later.

Since the main recovery target material in the monomer composition for synthesizing recycled plastics of the one embodiment is an aromatic diol compound, the dimethyl carbonate can be separately separated and recovered as the by-product from the monomer composition for synthesizing recycled plastics of the one embodiment.

The monomer composition for synthesizing recycled plastics of the one embodiment can be used as a raw material for preparing various recycled plastics (e.g., polycarbonate (PC)) which will be described later.

The monomer composition for synthesizing recycled plastics of the one embodiment may further include small amounts of other additives and solvents. Specific types of the additives or solvents are not particularly limited, and various materials widely used in the process of recovering the aromatic diol compound by a depolymerization of the polycarbonate-based resin can be applied without limitation.

The monomer composition for synthesizing recycled plastics of the one embodiment can be obtained by a method for preparing a monomer composition for synthesizing recycled plastics, which will be described later. That is, the monomer composition for synthesizing recycled plastics of one embodiment corresponds to the result obtained through various processes of filtration, purification, washing, and drying in order to secure only the aromatic diol compound, which is the main recovery target material, in high purity after the depolymerization reaction of the polycarbonate-based resin.

### 2. Method for preparing a monomer composition for synthesizing recycled plastic

According to another embodiment of the invention, there can be provided a method for preparing a monomer composition for synthesizing recycled plastic, the method comprising the steps of: subjecting a polycarbonate-based resin to a depolymerization reaction; separating a carbonate precursor from a depolymerization reaction product; and purifying the depolymerization reaction product from which the carbonate precursor has been separated, wherein the purifying the depolymerization reaction product from which the carbonate precursor has been separated comprises adding a hydrophilic reducing agent to the depolymerization reaction product from which the carbonate precursor has been separated; and adding an adsorbent to the depolymerization reaction product from which the carbonate precursor has been separated to perform adsorption purification, and then removing the adsorbent.

The present inventors confirmed through experiments that similarly to the method for preparing the monomer composition for synthesizing recycled plastic of the other embodiments, as a hydrophilic reducing agent adding step and an adsorption purification step by the adsorbent are included in the process of recycling a polycarbonate-based resin by chemical decomposition, the amount of organic solvent used in the adsorption purification step can be reduced, thereby achieving excellent color quality while increasing the efficiency of the process, and completed the present invention.

Specifically, the method for preparing a monomer composition for synthesizing recycled plastic according to the other embodiments may further include a step of pretreating the polycarbonate-based resin before the step of subjecting the polycarbonate-based resin to a depolymerization reaction.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate precursor. When it contains one carbonate repeating unit obtained by using only one aromatic diol compound and one carbonate precursor, a homopolymer can be synthesized. In addition, when one aromatic diol compound and two or more carbonate precursors are used as the monomer, or two or more aromatic diol compounds and one carbonate precursor are used, or one or more other diols is used in addition to the one aromatic diol compound and the one carbonate precursor to contain two or more carbonates, a copolymer can be synthesized. The homopolymer or copolymer can include all of lowmolecular compounds, oligomers, and polymers depending on the molecular weight range.

The polycarbonate-based resin can be applied regardless of various forms and types, such as a novel polycarbonate-based resin produced through synthesis, a recycled polycarbonate-based resin produced through a regeneration process, or polycarbonate-based resin waste.

A pretreatment step of the polycarbonate-based resin is carried out, thereby capable of increasing the efficiency of the process of recovering the aromatic diol compound and the carbonate precursor from the polycarbonate-based resin. Examples of the pretreatment step may include filtering, washing, drying, grinding, glycol decomposition, and the like. The specific method of each pretreatment step is not limited, and various methods widely used in the process of recovering the aromatic diol compound and the carbonate precursor from the polycarbonate-based resin can be applied without limitation.

However, an example of the pretreatment steps may include a step of dissolving the polycarbonate-based resin in an organic solvent and then filtering it. The organic solvent may include tetrahydrofuran, toluene, methylene chloride, chloroform, dimethyl carbonate, ethylmethyl carbonate, diethyl carbonate, dipropyl carbonate, or a mixture of two or more thereof.

Meanwhile, the method for preparing the monomer composition for synthesizing recycled plastics of the other embodiment may include a step of depolymerizing the polycarbonate-based resin.

During the depolymerization reaction of the polycarbonate-based resin, the depolymerization reaction may be carried out under acidic, neutral or basic conditions, and particularly, the depolymerization reaction may be carried out under basic (alkali) conditions. The type of the base is not particularly limited, and examples thereof include sodium hydroxide (NaOH) or potassium hydroxide (KOH). The base is a base catalyst acting as a catalyst, and has the economic advantages over organic catalysts, which are mainly used under mild conditions.

During the depolymerization reaction of the polycarbonate-based resin, the depolymerization reaction may be carried out by reacting a base in an amount of 0.5 moles or less, or 0.4 moles or less, or 0.3 moles or less, or 0.1 moles or more, or 0.2 moles or more, or 0.1 moles to 0.5 moles, or 0.1 moles to 0.4 moles, or 0.1 moles to 0.3 moles, or 0.2 moles to 0.5 moles, or 0.2 moles to 0.4 moles, or 0.2 moles to 0.3 moles relative to 1 mole of polycarbonate-based resin. When the polycarbonate-based resin is reacted with a base in an amount of more than 0.5 moles relative to 1 mole of the polycarbonate-based resin during depolymerization of the polycarbonate-based resin, there is a limit that impurities increase due to the effect of increasing the amount of alkali salt generated, so the purity of the target recovery material is reduced, and the economic efficiency of the catalytic reaction is reduced.

Further, the depolymerization reaction of the polycarbonate-based resin can be carried out in the presence of a solvent including ethanol. The present invention can stably obtain bisphenol A, which is a high-purity monomer, by decomposing a polycarbonate-based resin with a solvent including ethanol, and has the advantage that diethyl carbonate having a high added value can be further obtained as a reaction by-product.

The content of the ethanol may be 5 to 15 moles, or 8 to 13 moles relative to 1 mole of the polycarbonate-based resin. Since the ethanol has good solubility in bisphenol A, ethanol within the above range should be essentially contained. When the content of the ethanol is excessively reduced to less than 5 moles relative to 1 mole of the polycarbonate-based resin, it is difficult to sufficiently progress the alcohol decomposition of polycarbonate-based resin. On the other hand, when the content of ethanol is excessively increased to more than 15 moles relative to 1 mole of the polycarbonate-based resin, the economics of the process can be reduced due to excessive use of alcohol.

The solvent in which the depolymerization reaction of the polycarbonate-based resin proceeds may further include, in addition to ethanol, at least one organic solvent selected from the group consisting of tetrahydrofuran, toluene, methylene chloride, chloroform, dimethyl carbonate, ethylmethyl carbonate, diethyl carbonate, and dipropyl carbonate.

The organic solvent may include tetrahydrofuran, toluene, methylene chloride, chloroform, dimethyl carbonate, ethylmethyl carbonate, diethyl carbonate, dipropyl carbonate, or a mixture of two or more thereof.

More preferably, methylene chloride can be used as the organic solvent. When methylene chloride is used as an organic solvent to be mixed with the ethanol, there is an advantage that the dissolution properties in polycarbonate can be improved and the reactivity can be enhanced.

The content of the organic solvent may be 16 moles to 20 moles, or 16 moles to 18 moles relative to 1 mole of the polycarbonate-based resin. In addition, the content of the organic solvent may be 1.5 moles to 2 moles relative to 1 mole of ethanol. By mixing the polycarbonate-based resin, ethanol, and an organic solvent within the above range, there is an advantage that the depolymerization reaction of the polymer can proceed at a desired level.

Meanwhile, the temperature at which the depolymerization reaction of the polycarbonate-based resin proceeds is not particularly limited, but for example, the reaction may proceed at a temperature of 20°C to 100°C, or 50°C to 70°C. In addition, the depolymerization reaction of the polycarbonate-based resin may proceed for 1 hour to 30 hours, or 4 hours to 6 hours.

Specifically, the conditions are mild process conditions relative to the conventional pressurizing/high temperature process, and by performing stirring under the above conditions, the process can be performed in a mild process as compared to the pressurizing/high temperature process. In particular, when stirring at 50°C to 70°C for 4 to 6 hours, there is an advantage of obtaining the most efficient results in terms of reproducibility and stability.

That is, according to the present invention, by adjusting the type and mixing amount of the mixed solvent and the type and content of the base catalyst without using an organic catalyst, there is the advantage that a high-purity aromatic diol compound (e.g., bisphenol A) can be obtained under mild conditions without using a pressure/high temperature process, and diethyl carbonate, dimethyl carbonate, ethylmethyl carbonate can be obtained as the by-products by using an ethanol solvent.

More specifically, the step of subjecting a polycarbonate-based resin to a depolymerization reaction may comprise a first step of dissolving the polycarbonate-based resin in an organic solvent; and a second step of adding a catalyst solution containing ethanol and a base. In the first and second steps, the details of ethanol, organic solvent, base, and polycarbonate-based resin are the same as described above.

Further, the step of subjecting a polycarbonate-based resin to a depolymerization reaction may comprise a step of adding an antioxidant to the reactant containing the polycarbonate-based resin. Thereby, since the aromatic diol compound obtained during the depolymerization reaction of the polycarbonate-based resin is easily oxidized under basic conditions, an antioxidant can be added to the reaction solution.

The antioxidant may comprise at least one compound selected from the group consisting of sodium dithionite (Na₂S₂O₄, SDT), sodium metabisulfite(Na₂S₂O₅), and sodium sulfite(Na₂SO₃). That is, the antioxidant may comprise one type of sodium dithionite (Na₂S₂O₄, SDT), one type of sodium metabisulfite(Na₂S₂O₅), one type of sodium sulfite(Na₂SO₃), or a mixture of two or more types thereof.

The addition amount of the antioxidant is not particularly limited, but can be added, for example, from 0.05% by weight to 4% by weight based on the weight of the polycarbonate-based resin. Within the above range, it is effective in preventing oxidation, is advantageous in terms of cost, and does not lower the decomposition reaction rate.

The adsorbent may include at least one activated carbon selected from the group consisting of plant-based activated carbon, coal-based activated carbon, petroleum-based activated carbon, and waste activated carbon.

Meanwhile, the method for preparing the monomer composition for synthesizing recycled plastics of the other embodiment may further include a step of neutralizing the depolymerization reaction product with an acid, before the step of separating the carbonate precursor from the depolymerization reaction product.

For example, the alkali decomposition product of the polycarbonate-based resin includes an aromatic diol compound, or a salt thereof, but the main recovery target material of the present invention is an aromatic diol compound. Therefore, in the case of the salt of the aromatic diol compound obtained by alkaline decomposition, it can be converted into an aromatic diol compound through an additional acid neutralization process. That is, when the depolymerization reaction of the polycarbonate-based resin is an alkaline decomposition, it can undergo a neutralization reaction step with an acid.

The acid used in the neutralization reaction may be a strong acid, for example, hydrochloric acid (HCl). Due to the neutralization reaction by the strong acid, the pH can satisfy 4 or less or less than 2 upon completion of the neutralization reaction. The temperature during the neutralization reaction can be adjusted to 25°C or more and 100°C or less.

Further, if necessary, after proceeding the neutralization reaction step of the depolymerization reaction product by an acid, a step of removing residual impurities through filtration or absorption can be further performed. Specifically, the aqueous layer and the organic layer can be separated, and the organic layer can be filtered through vacuum filtration to recover the liquid containing the aromatic diol compound.

Meanwhile, the method for preparing the monomer composition for synthesizing recycled plastics of the other embodiment may include a step of separating a carbonate precursor from the depolymerization reaction product. Thus, the separated carbonate precursor may include diethyl carbonate.

For example, the depolymerization reaction product of the polycarbonate-based resin includes an aromatic diol compound or a salt thereof and a carbonate precursor. The details of the aromatic diol compound and the carbonate precursor include all of the contents mentioned above in the one embodiment.

The step of separating a carbonate precursor from a depolymerization reaction product may comprise a step of distilling the depolymerization reaction product under reduced pressure. Examples of the reduced pressure distillation conditions are not particularly limited, but in specific examples, performing a reduced pressure distillation of the depolymerization reaction product of the polycarbonate-based resin under the conditions wherein pressure is reduced at a pressure of 200 mbar to 300 mbar and a temperature of 20°C to 30°C, then at a pressure of 50 mbar to 150 mbar and a temperature of 25°C to 35°C, and then a pressure of 10 mbar to 50 mbar and a temperature of 35°C to 45°C. In addition, ethanol(EtOH) may be distilled off under the conditions wherein pressure is reduced at a pressure of 50 mbar to 150 mbar and a temperature of 25°C to 35°C. Further, the carbonate precursor can be separated and recovered under the conditions wherein pressure is reduced at pressure at a pressure of 10 mbar to 50 mbar and a temperature of 35°C to 45°C.

The separated carbonate precursor can be recycled without a separate separation and purification process, or can be recycled through separation and purification such as conventional extraction, adsorption, and drying, if necessary. Specific purification conditions are not particularly limited. As for the specific purification devices and methods, various well-known purification techniques can be applied without limitation.

Meanwhile, the method for preparing the monomer composition for synthesizing recycled plastics of the other embodiment may further include a step of purifying the depolymerization reaction product from which the carbonate precursor has been separated. Thereby, an aromatic diol compound, which is the main recovery material, is obtained, which corresponds to the monomer composition for synthesizing recycled plastics of the one embodiment.

Specifically, the purification step of the depolymerization reaction product from which the carbonate precursor has been separated may comprise a step of adding a hydrophilic reducing agent to the depolymerization reaction product from which the carbonate precursor has been separated; and a step of adding an adsorbent to the depolymerization reaction product from which the carbonate precursor has been separated to perform adsorption purification, and then removing the adsorbent.

In the purification step, the order of the step of adding a hydrophilic reducing agent and the adsorption purification step are not particularly limited, and it is irrelevant to proceed in any order, but for example, the purification step can be performed in the order of the adsorption purification step after the step of adding a hydrophilic reducing agent. The step of adding a hydrophilic reducing agent; and the adsorption purification step can proceed repeatedly at least once or more. As for the addition of specific reducing agents, adsorption devices and methods, various well-known purification techniques can be applied without limitation.

Meanwhile, before the step of adding a hydrophilic reducing agent to the depolymerization reaction product from which the carbonate precursor has been separated, the method may further comprise a redissolution step of adding a mixture of an organic solvent and an aqueous solvent to the depolymerization reaction product from which the carbonate precursor has been separated.

Specifically, in the washing step of the depolymerization reaction product from which the carbonate precursor has been separated, the depolymerization reaction product from which the carbonate precursor has been separated may contain an aromatic diol compound. However, since various impurities remain during the recovery process of obtaining the aromatic diol compound, washing can proceed in order to sufficiently remove these impurities and secure a high-purity aromatic diol compound.

Specifically, the washing step may include a step of washing with a solvent at a temperature of 10°C to 30°C, or 20°C to 30°C; and a step of washing with a solvent at a temperature of 40°C to 80°C, or 40°C to 60°C, or 45°C to 55°C. The temperature condition means the temperature inside a washing container at which washing with a solvent is performed. In order to maintain a high temperature deviating from a room temperature, various heating devices can be applied without limitation.

In the washing step, the step of washing with a solvent at a temperature of 10°C to 30°C may be performed first, and the step of washing with a solvent at a temperature of 40°C to 80°C may be performed later. Alternatively, the step of washing with a solvent at a temperature of 40°C to 80°C may be performed first, and the step of washing with a solvent at a temperature of 10°C to 30°C may be performed later.

More preferably, in the washing step, the step of washing with a solvent at a temperature of 10°C to 30°C may be performed first, and the step of washing with a solvent at a temperature of 40°C to 80°C may be performed later. Thereby, the corrosion of the reactor due to strong acid after the neutralization step can be minimized.

The step of washing with a solvent at a temperature of 10°C to 30°C; and the step of washing with a solvent at a temperature of 40°C to 80°C can proceed repeatedly at least once or more, respectively.

Further, if necessary, after proceeding the step of washing with a solvent at a temperature of 10°C to 30°C; and the step of washing with a solvent at a temperature of 40°C to 80°C, a step of removing the residual solvent through filtration may be further performed.

More specifically, the difference value between the temperature of the step of washing with a solvent at a temperature of 40°C to 80°C and the temperature of the step of washing with a solvent at a temperature of 10°C to 30°C may be 20°C to 50°C.

The difference value between the temperature of the step of washing with a solvent at a temperature of 40°C to 80°C and the temperature of the step of washing with a solvent at a temperature of 10°C to 30°C means a value obtained by subtracting the temperature of the step of washing with a solvent at a temperature of 10°C to 30°C from the temperature of the step of washing with a solvent at a temperature of 40°C to 80°C.

When the difference value between the temperature of the step of washing with a solvent at a temperature of 40°C to 80°C and the temperature of the step of washing with a solvent at a temperature of 10°C to 30°C decreases excessively to less than 20°C, it is difficult to sufficiently remove impurities.

When the difference value between the temperature of the step of washing with a solvent at a temperature of 40°C to 80°C and the temperature of the step of washing with a solvent at a temperature of 10°C to 30°C increases excessively to more than 50°C, severe conditions are formed to maintain extreme temperature conditions, which can reduce the efficiency of the process.

The solvent used in the washing step may include one of water, alcohol, and an organic solvent. As the organic solvent, tetrahydrofuran, toluene, methylene chloride, chloroform, dimethyl carbonate, ethylmethyl carbonate, diethyl carbonate, dipropyl carbonate, or a mixture of two or more thereof can be used.

The solvent used in the washing step can be used in a weight ratio of 1 part by weight or more and 30 parts by weight or less, or 1 part by weight or more and 10 parts by weight or less, based on 1 part by weight of the polycarbonate-based resin used in the depolymerization reaction.

More specifically, the solvent in the step of washing with a solvent at a temperature of 10°C to 30°C may be an organic solvent. Methylene chloride can be preferably used as the organic solvent. At this time, the organic solvent can be used in an amount of 1 part by weight or more and 10 parts by weight or less based on 1 part by weight of the polycarbonate-based resin.

Moreover, the solvent in the step of washing with a solvent at a temperature of 40°C to 80°C may be water. When water is used, impurities in the form of residual salts can be effectively removed. At this time, the solvent can be used in an amount of 1 part by weight or more and 10 parts by weight or less based on 1 part by weight of the polycarbonate-based resin.

Meanwhile, before the step of adding a hydrophilic reducing agent to the depolymerization reaction product from which the carbonate precursor has been separated, the method may further comprise a redissolution step of adding a mixture of an organic solvent and an aqueous solvent to the depolymerization reaction product from which the carbonate precursor has been separated. Through the step of adding a mixture of an organic solvent and an aqueous solvent to the depolymerization reaction product from which the carbonate precursor has been separated, the aromatic diol compounds included in the depolymerization reaction product from which the carbonate precursor has been separated may be redissolved in a mixture of an organic solvent and an aqueous solvent. Examples of the organic solvent include ethanol, and examples of the aqueous solvent include water.

The order of the washing step and the redissolving step is not particularly limited, and it is irrelevant to proceed in any order, but for example, the purification step may proceed in the order of the redissolving step after the washing step. The washing step; and the redissolving step can proceed repeatedly at least once or more. As for the specific washing and redissolving equipment and methods, various well-known purification techniques can be applied without limitation.

Specifically, the mixture of the organic solvent and the aqueous solvent may comprise 15% to 50% by weight of an organic solvent and 50% to 85% by weight of an aqueous solvent, based on the total weight of the mixture. When the aqueous solvent is reduced to less than 50% by weight based on the total weight of the mixture, there is a problem that the organic solvent is contained in a relatively large amount, and the process cost due to the use and recovery of the organic solvent increase, thereby reducing the efficiency of the process. On the other hand, when the aqueous solvent is increased to more than 85% by weight based on the total weight of the mixture, there is a problem that the solubility in the aromatic diol compound crystals contained in the depolymerization reaction product from which the carbonate precursor has been separated is lowered, and thus, redissolution is difficult to proceed sufficiently.

Meanwhile, the method for preparing a monomer composition for synthesizing recycled plastic according to the other embodiment may comprise a step of adding a hydrophilic reducing agent to a depolymerization reaction product from which the carbonate precursor has been separated.

The hydrophilic reducing agent has solubility in water and change colored materials, and can be used as a bleaching agent that removes a color. Thus, as the hydrophilic reducing agent is added, it is possible to enhance the colorless characteristic of the aromatic diol compound contained in the depolymerization reaction product from which the carbonate precursor has been separated.

Examples of the hydrophilic reducing agent are not particularly limited, and various hydrophilic reducing bleaches known in the art can be applied without limitation, and one example thereof is sodium dithionite (SDT).

Further, the addition amount of the hydrophilic reducing agent based on the weight of the depolymerization reaction product from which the carbonate precursor has been separated may be 0.1% by weight to 7% by weight, or 0.1% by weight to 6% by weight, or 0.1% by weight to 5% by weight, or 1% by weight to 7% by weight, or 1% by weight to 6% by weight, or 1% by weight to 5% by weight, or 3% by weight to 7% by weight, or 3% by weight to 6% by weight, or 3% by weight to 5% by weight, or 0.1% by weight to 3% by weight, or 1% by weight to 3% by weight,.

The depolymerization reaction product from which the carbonate precursor has been separated may include an aromatic diol compound. That is, the addition amount of the hydrophilic reducing agent may be 0.1% by weight to 7% by weight, or 0.1% by weight to 6% by weight, or 0.1% by weight to 5% by weight, or 1% by weight to 7% by weight, or 1% by weight to 6% by weight, or 1% by weight to 5% by weight, or 3% by weight to 7% by weight, or 3% by weight to 6% by weight, or 3% by weight to 5% by weight, or 0.1% by weight to 3% by weight, or 1% by weight to 3% by weight based on the weight of the aromatic diol compound contained in the depolymerization reaction product from which the carbonate precursor was separated.

When the addition amount of the hydrophilic reducing agent is excessively increased, the hydrophilic reducing agent is not sufficiently dissolved, or the side reaction makes it difficult to sufficiently realize the effect of improving the color characteristics. On the other hand, when the addition amount of the hydrophilic reducing agent is excessively reduced, it is difficult to sufficiently realize the effects of improving color characteristics by the hydrophilic reducing agent.

Meanwhile, in the step of adding an adsorbent to the depolymerization reaction product from which the carbonate precursor has been separated to perform adsorption purification, and then removing the adsorbent, an adsorbent may be brought into contact with the depolymerization reaction product.

Examples of the adsorbent may be activated carbon, charcoal, celite, or a mixture thereof. The activated carbon is a black carbon material having micropores produced by subjecting a raw material to a carbonization process at about 500°C and an activated carbon process at about 900°C, and examples thereof are not particularly limited, but for example, various activated carbons such as plant-based, coal-based, petroleum-based, waste-based activated carbons can be applied without limitation depending on the type of raw material.

In more specific examples, the plant-based activated carbon may include coconut activated carbon, wood activated carbon, and sawdust activated carbon. Further, the coal-based activated carbon may include lignite activated carbon, bituminous coal activated carbon, and anthracite activated carbon. Further, the petroleum-based activated carbon may include petroleum coke activated carbon and oil carbon activated carbon. Further, the waste activated carbon may include synthetic resin activated carbon and pulp activated carbon.

The adsorbent may include at least one activated carbon selected from the group consisting of plant-based activated carbon, coal-based activated carbon, petroleum-based activated carbon, and waste-based activated carbon. That is, the adsorbent may include plant-based activated carbon, coal-based activated carbon, petroleum-based activated carbon, waste-based activated carbon, or a mixture of two or more thereof.

More specifically, the adsorbent may include at least one activated carbon selected from the group consisting of coconut activated carbon, lignite activated carbon, anthracite activated carbon, and bituminous coal activated carbon. That is, the adsorbent may include coconut activated carbon, lignite activated carbon, anthracite activated carbon, bituminous coal activated carbon, or a mixture of two or more thereof.

In this manner, the method for preparing a monomer composition for synthesizing recycled plastics according to the other embodiments applies an adsorption purification step using an adsorbent during a depolymerization reaction in which a polycarbonate-based resin is recycled by chemical decomposition, not only an aromatic diol compound, which is a main synthetic target material in the present invention, can be secured in high purity, but also the content of impurities other than the aromatic diol compound can be significantly reduced.

Adsorption purification conditions by the adsorbent are not particularly limited, and various well-known adsorption purification conditions can be used without limitation. However, for example, the addition amount of the adsorbent may be 40% to 60% by weight relative to the polycarbonate-based resin, and the adsorption time may be 0.1 hour to 5 hours, and the adsorption method may be a stirring adsorption or an adsorption tower for Lab.

Meanwhile, after the step of adding an adsorbent to the depolymerization reaction product from which the carbonate precursor has been separated to perform adsorption purification, and then removing the adsorbent, the method may further comprise a step of recrystallizing the depolymerization reaction product from which the carbonate precursor has been separated.

In the recrystallization step of the depolymerization reaction product from which the carbonate precursor has been separated, a high-purity aromatic diol compound can be secured by sufficiently removing various impurities contained in the depolymerization reaction product from which the carbonate precursor has been separated.

Specifically, the recrystallization step may include a step of adding a recrystallization solvent to the depolymerization reaction product from which the carbonate precursor has been separated to perform recrystallization. Through the step of adding a recrystallization solvent to the depolymerization reaction product from which the carbonate precursor has been separated to perform recrystallization, the solubility of the aromatic diol compound or its salt contained in the depolymerization reaction product is increased, and thus, crystals, or impurities interposed between crystals can be dissolved with a solvent to the maximum, and further, since the dissolved aromatic diol compound has poor solubility relative to impurities, it can be easily precipitated into aromatic diol compound crystals through the difference in solubility when the temperature is lowered subsequently. Water can be used as the recrystallization solvent.

More specifically, the step of recrystallizing the depolymerization reaction product from which the carbonate precursor has been separated may comprise a step of adding 5 parts by weight to 25 parts by weight of a recrystallization solvent based on 1 part by weight of the aromatic diol compound contained in the depolymerization reaction product. When the recrystallization solvent is used in an excessively small amount, the temperature for dissolving the aromatic diol compound contained in the depolymerization reaction product from which the carbonate precursor has been separated becomes too high, which thus deteriorates in the process efficiency, and it is difficult to remove impurities through recrystallization. On the other hand, when a recrystallization solvent is used in an excessive amount, the solubility of the aromatic diol compound contained in the depolymerization reaction product from which the carbonate precursor has been separated becomes too high, and thus, the yield of the aromatic diol compound recovered after recrystallization is reduced, and the process efficiency can be reduced due to the use of large amounts of solvent.

If necessary, after proceeding the recrystallization step of the depolymerization reaction product from which the carbonate precursor has been separated, a step of removing residual impurities through filtration or adsorption can be further performed.

In addition, if necessary, after the recrystallization step, the method may further include a drying step. The remaining solvent can be removed by the drying, and the specific drying conditions are not particularly limited, but for example, the drying can be performed at a temperature of 10°C to 100°C, or 10°C to 50°C. As for the specific drying equipment and method used in the drying, various well-known drying techniques can be applied without limitation.

### 3. Recycled Plastic

According to another embodiment of the invention, a recycled plastic comprising a reaction product of the monomer composition for synthesizing recycled plastic of the one embodiment and a comonomer can be provided.

The details of the monomer composition for synthesizing recycled plastic of the one embodiment include all the contents described above in the one embodiment and the other embodiment.

Examples corresponding to the recycled plastic are not particularly limited, and various plastics synthesized from aromatic diol compounds such as bisphenol A as a monomer can be applied without limitation, and a more specific example may be a polycarbonate-based resin.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate precursor. When it contains one carbonate repeating unit obtained by using only one aromatic diol compound and one carbonate precursor, a homopolymer can be synthesized. In addition, when one aromatic diol compound and two or more carbonate precursors are used as the monomer, or two or more aromatic diol compounds and one carbonate precursor are used, or one or more other diols is used in addition to the one aromatic diol compound and the one carbonate precursor to contain two or more carbonates, a copolymer can be synthesized. The homopolymer or copolymer can include all of lowmolecular compounds, oligomers, and polymers depending on the molecular weight range.

More specifically, in the recycled plastic containing the reaction product of the monomer composition for synthesizing the recycled plastic and the comonomer of the one embodiment, a carbonate precursor can be used as the comonomer. Specific examples of the carbonate precursor include phosgene, triphosgene, diphosgene, bromophosgene, dimethyl carbonate, diethyl carbonate, dibutyl carbonate, dicyclohexyl carbonate, diphenyl carbonate, ditolyl carbonate, bis(chlorophenyl)carbonate, m-cresyl carbonate, dinaphthyl carbonate, bis(diphenyl) carbonate or bishaloformate.

Examples of the reaction process of the monomer composition for synthesizing recycled plastic and the comonomer are not particularly limited, and various well-known polycarbonate preparation methods can be applied without limitation.

However, in one example of the polycarbonate preparation method, a polycarbonate preparation method including the step of polymerizing a composition containing a monomer composition for synthesizing recycled plastic and a comonomer can be used. At this time, the polymerization can be carried out by interfacial polymerization, and during interfacial polymerization, polymerization reaction is possible at normal pressure and low temperature, and the molecular weight is easy to control.

The polymerization temperature may be 0°C to 40°C, and the reaction time may be 10 minutes to 5 hours. In addition, the pH during the reaction may be maintained at 9 or more or 11 or more.

The solvent that can be used for the polymerization is not particularly limited as long as it is a solvent used for polymerization of polycarbonate in the art, and as an example, halogenated hydrocarbons such as methylene chloride and chlorobenzene can be used.

Moreover, the polymerization can be carried out in the presence of an acid binder. As the acid binder, an alkali metal hydroxide such as sodium hydroxide or potassium hydroxide, or an amine compound such as pyridine can be used.

Further, in order to adjust the molecular weight of the polycarbonate during the polymerization, polymerization can be performed in the presence of a molecular weight modifier. An alkylphenol having 1 to 20 carbon atoms may be used as the molecular weight modifier, and specific examples thereof include p-tert-butylphenol, p-cumylphenol, decylphenol, dodecylphenol, tetradecylphenol, hexadecylphenol, octadecylphenol, eicosylphenol, docosylphenol or triacontylphenol. The molecular weight modifier can be added before, during or after the initiation of polymerization. The molecular weight modifier may be used in an amount of 0.01 to 10 parts by weight, or 0.1 to 6 parts by weight, based on 100 parts by weight of the aromatic diol compound, and a desired molecular weight can be obtained within this range.

In addition, in order to promote the polymerization reaction, a reaction accelerator such as a tertiary amine compound, a quaternary ammonium compound, or a quaternary phosphonium compound, including triethylamine, tetra-n-butylammonium bromide, or tetra-n-butylphosphonium bromide can be further used.

### 4. Molded Product

According to another embodiment of the invention, a molded article comprising the recycled plastic of the other embodiment can be provided. The details of the recycled plastic includes all the contents described above in the other embodiments.

The molded article can be obtained by applying the recycled plastic to various known plastic molding methods without limitation. As an example of the molding method, injection molding, foam injection molding, blow molding, or extrusion molding may be mentioned.

Examples of the molded article are not particularly limited, and can be applied to various molded articles using plastic without limitation. Examples of the molded article include automobiles, electrical and electronic products, communication products, daily necessities, building materials, optical components, exterior materials, and the like.

The molded article may further include one or more additives selected from the group consisting of an antioxidant, a plasticizer, an antistatic agent, a nucleating agent, a flame retardant, a lubricant, an impact enhancer, an optical brightener, an ultraviolet absorber, a pigment and a dye, if necessary, in addition to the recycled plastic of the other embodiments,

An example of the manufacturing method of the molded article may include a step of mixing the recycled plastic of the other embodiment and an additive well using a mixer, extrusion-molding the mixture with an extruder to produce pellets, drying the pellets, and then injecting them with an injection molding machine.

### [Advantageous Effects]

According to the present invention, a monomer composition for synthesizing recycled plastic that can realize excellent color quality even though it is recovered through recycling by chemical decomposition of a polycarbonate-based resin, and allows improvement of efficiency in the recovery process, a preparation method thereof, and a recycled plastic and a molded product using the same can be provided.

### [DETAILED DESCRIPTION OF THE EMBODIMENTS]

Hereinafter, the present invention will be explained in detail with reference to the following examples. However, these examples are for illustrative purposes only, and the scope of the present invention is not limited thereto.

### <EXAMPLE, COMPARATIVE EXAMPLE AND REFERENCE EXAMPLE: Preparation of recycled bisphenol A monomer composition>

### Example 1

(1. Decomposition step) 1 mol of pretreated waste polycarbonate(PC) was dissolved in 17 mol of methylene chloride(MC), and then added together with 11 mol of ethanol (EtOH) and 0.25 mol of sodium hydroxide (NaOH) to a 3L high-pressure reactor, and 2.5g of sodium dithionite (Na₂S₂O₄, SDT) (1 wt.% relative to PC weight) was added thereto, and stirred at 60°C for 6 hours to proceed PC depolymerization reaction.
(2. Neutralization stage) The mixture containing the bisphenol A was neutralized using 0.25 mole of 1N hydrochloric acid (HCl) at 20~30°C, the aqueous layer and the organic layer were separated, and the organic layer was filtered through vacuum filtration to obtain a liquid containing bisphenol A.
(3. Distillation step) Then, methylene chloride (MC) was distilled off at 250 mbar and 20°C, ethanol (EtOH) was distilled off at 80 mbar and 30°C, and then distilled under reduced pressure at 30 mbar and 40°C to diethyl carbonate (DEC) as a by-product.
(4. Washing step) Then, the residue from which diethyl carbonate (DEC) was removed was first washed by using methylene chloride (MC) in an amount of 1 times the mass of PC used at 20 to 30°C, and then filtered under vacuum.
(5. Redissolving step) 5 g of ethanol and 15 g of water were added to 6 g of the washed bisphenol A, and redissolved at room temperature.
(6. Reducing agent adding step) Then, 0.06 g (1 wt.% of bisphenol A) of sodium dithionite (Na₂S₂O₄, SDT) was added to the solution and dissolved.
(7. Adsorption step) Then, 3 g of lignite activated carbon as an adsorbent was added to the solution, and stirred for 10 minutes. Then, activated carbon was primarily removed using a sieve filter (75 *µ*m), and residual activated carbon was secondarily removed using a syringe filter (0.45 *µ*m).
(8. Recrystallization step) Then, 75 g of water was slowly added while stirring to recrystallize bisphenol A, and then then filtered to obtain a solid.
(9. Drying step) After that, it was dried in a vacuum oven at 30 to 50°C to prepare a recycled bisphenol A monomer composition.

### Example 2. Comparative Examples 1 to 3. and Reference Examples 1 and 2

The recycled bisphenol A monomer compositions of Example 2, Comparative Examples 1 to 3, and Reference Examples 1 and 2 were prepared in the same manner as in Example 1, except that the process conditions were changed as shown in Table 1 below.

**Table 1**

| Category | 1. Decomposition step | 5. Redissolving step | 6. Reducing agent adding step | 7. Adsorption step | 8. Recrystallization step |
|---|---|---|---|---|---|
| | Amount of SDT added (wt.% based on PC weight) | Amount of ethanol/water added | Amount of SDT added (wt.% based on bisphenol A weight) | Amount of adsorbent added | Amount of water added |
| Example 1 | 2.5g(1 wt.%) | 15g/15g (ethanol/water) | 0.06g (1 wt.%) | 3g | 75g |
| Example 2 | 2.5g(1 wt.%) | 15g/15g (ethanol/water) | 0.3g (5 wt.%) | 3g | 75g |
| Comparative Example 1 | 2.5g(1 wt.%) | 15g/15g (ethanol/water) | 0g (0 wt.%) | 0g | 75g |
| Comparative Example 2 | 2.5g(1 wt.%) | 15g/15g (ethanol/water) | 0.3g (5 wt.%) | 0g | 75g |
| Comparative Example 3 | 2.5g(1 wt.%) | 15g/15g (ethanol/water) | 0g (0 wt.%) | 3g | 75g |
| Reference Example 1 | 2.5g(1 wt.%) | 30g/0g (ethanol/water) | 0g (0 wt.%) | 3g | 180g |
| Reference Example 2 | 2.5g(1 wt.%) | 15g/15g (ethanol/water) | 0.6g (10 wt.%) | 3g | 75g |

### <Experimental Example>

The physical properties of the recycled bisphenol A monomer compositions obtained in Examples, Comparative Examples and Reference Examples were measured by the following method, and the results are shown in Table 2 below.

### 1. APHA Color

The APHA Color of the recycled bisphenol A monomer composition was measured according to the ASTM D 1209 test method using HunterLab UltraScan PRO Spectrophotometer device.

### 2. Color coordinates (L*, a*, b*)

The color coordinates of the recycled bisphenol A monomer composition was measured in reflection mode using a HunterLab UltraScan PRO Spectrophotometer.

**[Table 2]**

| Measurement results of Experimental Examples | | | | |
|---|---|---|---|---|
| Category | APHA Color | L* | a* | b* |
| Example 1 | 28 | 95.6 | -0.3 | 1.7 |
| Example 2 | 22 | 96.7 | -0.6 | 1.6 |
| Comparative Example 1 | 100 | 94.8 | 1.5 | 5.0 |
| Comparative Example 2 | 89 | 95.2 | 1.2 | 4.6 |
| Comparative Example 3 | 34 | 96.1 | 0 | 2.0 |
| Reference Example 1 | 21 | 96.5 | -0.2 | 1.3 |
| Reference Example 2 | 44 | 96.2 | -0.8 | 3.0 |

As shown in Table 2, the recycled bisphenol A monomer compositions obtained in Examples 1 and 2 exhibited color coordinates L* of 95.6 to 96.7, a* of -0.6 to -0.3, and b* of 1.6 to 1.7, and APHA Color values were also measured between 22 and 28, which exhibited excellent optical properties. In contrast, the recycled bisphenol A monomer compositions obtained in Comparative Examples 1 to 3 exhibited color coordinates L* of 94.8 to 96.1, a* of 0 to 1.5, and b* of 2.0 to 5.0, and APHA Color values were measured between 34 and 100, which exhibited poor optical properties as compared to those of Examples.

On the other hand, the recycled bisphenol A monomer composition obtained in Reference Example 1 exhibited optical properties at the same level as in Examples, but in order to achieve this, there was a problem of reduced efficiency in the process as excess water had to be used at 180 g in the recrystallization step as shown in Table 1 above.

The recycled bisphenol A monomer composition obtained in Reference Example 2 exhibited a color coordinate b* of 3.0 and an APHA color value was measured at 44, which exhibited poor optical properties as compared with Examples.

## Claims

1. A monomer composition for synthesizing recycled plastic,
which comprises an aromatic diol compound,
wherein a color coordinate b* is 1.8 or less,
wherein an APHA Color value measured according to ASTM D 1209 is 30 or less, and
wherein the monomer composition is recovered from a polycarbonate-based resin.

2. The monomer composition for synthesizing recycled plastic according to claim 1 wherein:
the monomer composition for synthesizing recycled plastic has a color coordinate L* of 95.5 or more.

3. The monomer composition for synthesizing recycled plastic according to claim 1 wherein:
the monomer composition for synthesizing recycled plastic has a color coordinate a* of -0.7 to -0.2.

4. The monomer composition for synthesizing recycled plastic according to claim 1 wherein:
the aromatic diol compound includes bisphenol A.

5. The monomer composition for synthesizing recycled plastic according to claim 1 wherein:
the aromatic diol compound is recovered from a polycarbonate-based resin.

6. The monomer composition for synthesizing recycled plastic according to claim 1 wherein:
the monomer composition for synthesizing recycled plastic further comprises a hydrophilic reducing agent.

7. A method for preparing a monomer composition for synthesizing recycled plastic, the method comprising the steps of:
subjecting a polycarbonate-based resin to a depolymerization reaction;
separating a carbonate precursor from a depolymerization reaction product; and
purifying the depolymerization reaction product from which the carbonate precursor has been separated,
wherein the purifying the depolymerization reaction product from which the carbonate precursor has been separated comprises,
adding a hydrophilic reducing agent to the depolymerization reaction product from which the carbonate precursor has been separated; and
adding an adsorbent to the depolymerization reaction product from which the carbonate precursor has been separated to perform adsorption purification, and then removing the adsorbent.

8. The method for preparing a monomer composition for synthesizing recycled plastic according to claim 7, wherein:
the hydrophilic reducing agent is sodium dithionite.

9. The method for preparing a monomer composition for synthesizing recycled plastic according to claim 7, wherein:
the amount of the hydrophilic reducing agent added is 0.1% by weight to 7% by weight, based on the weight of the depolymerization reaction product from which the carbonate precursor has been separated.

10. The method for preparing a monomer composition for synthesizing recycled plastic according to claim 7, wherein:
before the adding a hydrophilic reducing agent to the depolymerization reaction product from which the carbonate precursor has been separated,
the method further comprises a redissolution step of adding a mixture of an organic solvent and an aqueous solvent to the depolymerization reaction product from which the carbonate precursor has been separated.

11. The method for preparing a monomer composition for synthesizing recycled plastic according to claim 10, wherein:
the mixture contains 15% by weight to 50% by weight of an organic solvent and 50% by weight to 85% by weight of an aqueous solvent based on the total weight of the mixture.

12. The method for preparing a monomer composition for synthesizing recycled plastic according to claim 7, wherein:
the subjecting a polycarbonate-based resin to a depolymerization reaction comprises,
adding an antioxidant to the reactant containing the polycarbonate-based resin.

13. The method for preparing a monomer composition for synthesizing recycled plastic according to claim 12, wherein:
the antioxidant comprises at least one compound selected from the group consisting of sodium dithionite, sodium metabisulfite, and sodium sulfite.

14. The method for preparing a monomer composition for synthesizing recycled plastic according to claim 7, wherein:
before the adding a hydrophilic reducing agent to the depolymerization reaction product from which the carbonate precursor has been separated,
the method further comprises washing the depolymerization reaction product from which the carbonate precursor has been separated, and
wherein the washing comprises washing with a solvent at a temperature of 10°C to 30°C; and washing with a solvent at a temperature of 40°C to 80°C.

15. The method for preparing a monomer composition for synthesizing recycled plastic according to claim 7, wherein:
the depolymerization reaction of a polycarbonate-based resin,
proceeds in the presence of a solvent including ethanol.

16. The method for preparing a monomer composition for synthesizing recycled plastic according to claim 7, wherein:
the separating a carbonate precursor from a depolymerization reaction product comprises,
distilling the depolymerization reaction product under reduced pressure.

17. The method for preparing a monomer composition for synthesizing recycled plastic according to claim 7, wherein:
after the adding an adsorbent to the depolymerization reaction product from which the carbonate precursor has been separated to perform adsorption purification, and then removing the adsorbent,
the method further comprises recrystallizing the depolymerization reaction product from which the carbonate precursor has been separated.

18. The method for preparing a monomer composition for synthesizing recycled plastic according to claim 17, wherein:
the recrystallizing the depolymerization reaction product from which the carbonate precursor has been separated comprises,
adding 5 parts by weight to 25 parts by weight of a recrystallization solvent based on 1 part by weight of the aromatic diol compound contained in the depolymerization reaction product.

19. A recycled plastic comprising a reaction product of the monomer composition for synthesizing recycled plastic of claim 1 and a comonomer.

20. A molded product comprising the recycled plastic of claim 19.
